# EUROPEAN PATENT APPLICATION

(11) **EP 3 494 875 A1**
(43) Date of publication of application: **12.06.2019**
(21) Application number: 17206393.5
(22) Date of filing: 11.12.2017
(51) Int. Cl.: A61B 5/024, A61B 5/0408, A61B 5/0478, A61B 5/0492, A61N 1/04

(54) **SENSOR DEVICE FOR DETECTING AN ELECTRICAL SIGNAL**

(71) Applicant: ETH Zurich, 8092 Zurich (CH)
(72) Inventor: VÖRÖS, Janos, 8050 Zurich (CH); PETERS, Christian, 8005 Zürich (CH); STAUFFER, Flurin, 8342 Wernetshausen (CH); THIELEN, Moritz, 8052 Zürich (CH); CHARDONNENS, Séverine, 8004 Zürich (CH); BACHMANN, Simon, 8006 Zürich (CH)

(57) **Abstract**

The invention concerns a sensor device designed for contacting the skin of a user and detecting an electrical signal comprising:
- two main faces, a upper face and a lower face opposed to the lower face, the lower face being designed for being placed toward the skin, the upper face being designed for transmitting the signal,
- the lower face comprising an array of protrusions with micrometre dimensions, said protrusions protruding from the lower side toward the skin when the sensor is placed on the skin.

The array further comprises a conductive portion and a non-conductive portion, said non-conductive portion surrounding said conductive portion, so that when the sensor is placed on the skin. The non-conductive portion allows maintaining the contact between the conductive portion and the skin to ensure that the conductive portion is capable of detecting the signal.

The invention also concerns a method for manufacturing a sensor device.

## Description

### Field of the invention

The present invention concerns a sensor device and a method for manufacturing a sensor device.

### Description of related art

Medical devices for detecting, measuring and monitoring biological signal of a user, such as electrical signal, generally consist of two units: a sensor unit placed on the skin of the user connected to a computing unit capable of processing the measured signal.

Such medical devices can be used punctually for a short term measurement, for instance an electrography (ECG), electroencephalography (EEG) or electromyography (EMG) measurement during a medical consultation.

Alternatively, there is also a need for long term and continuous monitoring, notably for diagnosing certain coronary artery diseases that required ECG data over hours, or EEG setting for home monitoring for instance sleep monitoring to detect brain damages related diseases.

The document "Enhanced Skin Adhesive Patch with Modulus-Tunable Composite Micropillars", K.Y.Suh et al., Adv. Healthcare Mater., 2013, 2, 109-113, illustrates a known solution comprising an electrode and a gel that interfaces the contact between the electrode and the skin. The gel ensures sufficient contact area, sufficient hydration of the skin and good electrical contact. As long as the gel is operational, this solution allows providing reliable signals. However, the gel-based electrode cannot be worn for a long period of time due to skin irritation and drying of the electrolyte gel. Therefore, gel-based electrode are not suitable for long term monitoring. Additionally, gel-based electrodes cannot be used for reliable underwater recordings as the electrolyte gel will dissolve in the surrounding liquid.

Over the past years, an alternative technology comprising dry electrode has been developed to overcome the limitation of the gel-based electrode. Dry electrodes ensure comfort over a longer period of time but face their own challenges: as they do not work with electrolyte gel, the impedance is high before the sweat can act as electrolyte. They need an optimized surface architecture and softness in order to comply with the skin to lower the impedance and be robust against motion artefacts.

The document "Bioinspired, Highly Stretchable, and Conductive Dry Adhesives Based on 1D/2D Hybrid Carbon Nanocomposites for All-in-One ECG Electrodes", Jeon et al., ACS Nano, 2016, 10, 4770-4778 describes a dry electrode wherein the contact side of the electrode is structured with pillars and foot for improving the contact of the electrode on the skin, resulting in a better signal detection. The microstructures pillars surface toward the skin are made of carbon nanotubes. The dry electrode is fabricated by moulding the electrode in a specifically designed silicon mould. However, the solution disclosed in this documents is limited to experimental uses in a laboratory setting. The raw materials used in this process are expensive and the process is complex which represent an important draw back for industrialised setting. Additionally, such electrode present a low electrical conductivity due to the carbon based fillers. As a result, the electrodes need a large skin contact area that limits the special resolution of the signal to an extend that prevents clinical evaluation.

Additionally, dry electrodes disclosed in Jeon *et al*. are not suitable for dynamic wet environments because the microstructure conductive surface is not isolated so that in a wet environment, the detection of the signal is disturbed.

Regarding the manufacturing process, in Jeon *et al.* there is a strong strain on the microstructured pillars during the demoulding of the electrode, in particular on the foot. The foot is made of soft material that can be damaged when the foot is squeezed out of the stiff mould typically made of cured silicon for instance. As a results, the risk of microcrack formation and full rupture on the microstructure is very high, so that the dry electrode is very difficult to manufacture according to the disclosed process.

Therefore, there is a need for a sensor device that allows overcoming, at least partially, the limitations of the existing solutions.

### Brief summary of the invention

According to the invention, these aims are achieved by means of a sensor device designed for contacting the skin of a user and detecting an electrical signal, the device comprising:
- two main faces, an upper face and a lower face opposed to the upper face, the lower face being designed for being placed toward the skin, the upper face being designed for transmitting the signal,
- the lower face comprising an array of protrusions with micrometer dimensions, said protrusions protruding from the lower face toward the skin when the sensor is placed on the skin,
characterized in that the array comprises a conductive portion and a non-conductive portion, said non-conductive portion surrounding said conductive portion, so that when the sensor is placed on the skin, the non-conductive portion allows maintaining the contact between the conductive portion and the skin to ensure that the conductive portion is capable of detecting the signal.

The sensor device comprises a lower face and an upper face, said lower face being in charge of detecting the electrical signal from the skin. To that end the lower face comprises a conductive portion with an array of protrusions made of conductive material. The protrusions point towards the skin so that when the device is on the skin, the distal ends of the protrusions contact the skin and allow detection of the signal.

The array of the lower face also comprises a non-conductive portion that surrounds the conductive portion, so that the array is constituted by both non-conductive and conductive material. Advantageously, the protrusions provide an attachment to the skin which is comfortable as it does not rely on chemical attachment for instance via conventional sticky textile of acrylate derivatives, or silicon derivative. The sensor device can therefore be attached and reattached several times at the same location on the skin without irritating the skin and without losing adhesive properties as adhesion relies on geometrical attributes of the protrusions.

The protrusions also allow an improved contact between the lower surface and the skin. The sensor device, and in particular the protrusions, are made of soft and elastic polymer so that the sensor device can follow the motions and the surface variations of the skin while maintaining a remarkable signal detection. For instance, soft material are Polydimethylsiloxane (PDMS) and its derivatives.

Therefore, the protrusions array comprises a conductive portion and a non-conductive portion that allows increasing the adhesion on the skin together with an improved detection of the signal.

Thanks to the protrusions array, the sensor device has a relevant impedance value without using the gel and due to the adhesive properties of the protrusions, in particular the conductive portion, that reduces the motion artifact in the signal therefore enabling signal quality suitable for clinical uses.

The softness of the conductive material also helps providing a sensor device with relevant impedance value. In particular, motion artefact is reduced when the contact between skin and conductive portion stays intact, even during movement of the skin of user.

Advantageously, the non-conductive portion surrounds the conductive portion, in other words the non-conductive portion extends radially from the conductive portion, the conductive portion is embedded in the non-conductive portion. The conductive portion is isolated from the exterior environment by the non-conductive portion. For instance, the conductive portion is in a central area of the lower face (i.e. conductive area), whereas the non-conductive portion is in a peripheral area (i.e. non-conductive area) that surrounds said central area. The non-conductive portion acts as a barrier for the conductive portion, such as insulator barrier, electrical insulator, or water insulator, hydrophobic water barrier, thermal insulator, or adhesion enhancer.

Advantageously, for instance, the non-conductive portion water insulates the conductive portion, so that the sensor device is suitable for uses in wet environment, for instance when the user is swimming, diving or showering.

The conductive portion and the non-conductive portion can have various shapes. In an embodiment, the array comprises a central conductive portion surrounded by a peripheral non-conductive portion. In one embodiment, the conductive portion and the non-conductive portion are delineated by two circular surfaces, said areas being concentric on the surface of the lower face. Alternatively, the conductive portion and the non-conductive portion are delineated by polygonal surfaces, rectangular surfaces, or circular surfaces.

In one embodiment, the protrusions comprise a rod attached to the lower face, and a feet at the distal end of said rod, the feet having a diameter superior to the diameter of the rod. Advantageously, the feet allows increasing the adhesion of the sensor device on the skin. In this embodiment, the protrusions have a mushroom or gecko or grasshopper shape.

In an embodiment, the upper face further comprises a connector connected with the lower face, said connector allowing guiding the signal from the lower face to the upper face. For instance, the connector is a connective bridge extending from the conductive portion and embedded in the non-conductive portion toward the upper face. The connector is preferably designed for being connected with an external device, for instance a read out device.

In one embodiment, the upper face comprises interlocking means designed for interlocking the sensor device with an external device, for instance a read out device. The interlocking means are used for securing the connection between the sensor device and an external device, for instance a read out device. In one embodiment, said interlocking means are made of conductive material, so that they act both as interlocking means and connector.

In an embodiment, the interlocking means comprise a plurality of extensions extending from the upper face. For instance, the extensions have micrometer dimensions, such as a mushroom or gecko shape, or shapes and dimensions of a foot of grasshopper's insects or foot of other insects capable of dry adhesion. Conventional attachment systems for attaching the sensor device to an external device, for instance snapjoint, are known to stress or hurt the internal structure of the sensor device. Microstructures, such as the extensions, provide a very convenient and soft attachment system in a way that the force needed for reversibly attaching the sensor device does not hurt the internal structure of the sensor device. Interlocking means, such as gecko shaped feet or random microstructure acting as velcro, interlock when forces are applied parallel to the surface of the upper face, and detached when forces are applied transversal to the surface of the upper means.

In one embodiment the extensions are in electrical connection with the conductive portion of the lower part.

In one embodiment, the extensions extending from the upper face have similar geometries as the protrusions protruding from the lower face so that the sensor device displays a doubled sided microstructure.

In an embodiment, the upper face with the connector pad made of conductive material and the lower face with the conductive portion are mirror image of each other. In this embodiment, the sensor device the upper face and the lower face are identical, so that the sensor device is double sided is reversible, i.e. the device can be flipped over, the detection face (toward skin) become the connector face and vice versa, each face being bi functional. The advantage can be that there is no mechanical mismatch between the upper face and the lower face as it is made of the same material. Another advantage is when the geometry of the connector at the device is critical for it's function, it is possible to specifically design the requested geometry with the method according to the present invention.

In another embodiment, the ratio of conductive versus non-conductive extension or protrusions is different on the upper side and on the lower side. It is possible to tune the ratio or density of extensions or protrusions on each of the side to facilitate the connection of the upper side, or to increase the detection area on the lower side. It is also possible to provide the upper side with a first design of protrusion or extension with a specific aspect ratio (the aspect ratio being the ratio of the length/diameter of the protrusion) and the lower side with a different design of protrusion with a different aspect ratio, for instance thicker or longer. For instance, the first design of the extensions can interlock better whereas the second design of the protrusion have a better contact on the skin.

In one embodiment, the conductive portion comprises conductive nanoparticles or conductive microparticles, preferably with particles size between 0.1 nm and 10µm, preferably between 0.1 nm and 3500 nm, for example silver (Ag) nanoparticles, silver (Ag) microparticles or any other metal based micro- or nanoparticles suitable for a sensor device, carbon nanotubes, metal nano-wires.

In one embodiment, the conductive material is based on non-conductive material mixed with conductive microparticles or nanoparticles.

In an embodiment, the sensor device further comprises conductive wire connected to the conductive portion to guide the signal.

In an embodiment, at least part of the sensor device, in particular the conductive portion and/or the non-conductive portion, is made of soft material, for example a soft material with a softness in the range of the human skin. The elasticity of the soft material improves the contact between the sensor device and the skin. The elasticity also ensures that movement of the skin is mechanically taken up by the electrodes such that there is no contact loss between the electrode and the skin due to movement. Contact loss results in motion artifacts in the signal.

The invention also concerns a method for manufacturing a sensor device designed for contacting the skin of a user and detecting an electrical signal, the method comprising:
- i) Providing a mould for moulding the sensor device, the mould being constituted with a substrate designed for being dissolvable in a determined developer solution, the substrate comprising an array of recesses etched in said substrate;
- ii) Depositing a conductive material and/or a non-conductive material into the recesses of the array;
- iii) Dissolving the mould with the determined developer solution to release the sensor device.

Once the material, conductive and/or non-conductive, is moulded, the mould is removed by dissolution. The mould is engraved in the substrate and said substrate is made of a material dissolvable in a determined developer solution. For instance, the substrate is a sacrificial layer designed for being removed by dissolution in a sacrificial layer developer solution. When the conductive and/or non-conductive material is in the recesses of the mould, there is a solid-solid contact between the material (conductive or non-conductive) and the mould providing a strain on the moulded material. Once the mould is dissolved, the solid-solid contacts with to a solid-fluid contact as the mould is dissolved in the developer solution, so that the strain on the moulded material is removed. Hence, the demoulding of the sensor device occurs in a strain free manner, in particular on the protrusions formed in the recesses. This solution allows avoiding the micro cracks observed when the sensor device is demoulded from a hard mould.

Advantageously, the strain free demoulding process allows using soft or very soft material for the protrusions array. Soft of very soft material based sensor device, for instance electrode, allows providing a sensor device meeting the sensitivity requirements for clinical uses, without the need of electrolyte gel.

Generally, the mould to be dissolved is fabricated on a flat base material, typically a wafer. Advantageously, once the mould has been dissolved and the moulded sensor device removed, the wafer can be re-used to limit the cost of the fabrication process.

The existing methods are limited to moulding of conical shaped microstructure that prevents the material to be stuck in the mould during demoulding. Advantageously, dissolving the mould provides reliable method for demoulding soft and elastic material based microstructure because the material do not stay stuck in the mould so that the shape of the microstructure is not limited to conical shape.

Soft material or very soft material can be defined as materials that can be easily deformed by thermal stresses or thermal fluctuations at about room temperature. A elastic material can be defined as a material with high elasticity (Young's modulus-range), high tensile, compressive, shear and fatigue strength, high toughness and low hardness

In one embodiment, the method allows manufacturing sensor device with conductive material or non-conductive material.

In an embodiment, the method allows manufacturing sensor device with conductive material and nonconductive material.

In an embodiment, the method further comprises :
- Fabricating the mould by etching away selectively the substrate to etch the recesses, each recess comprises a cylindrical portion attached the lower face and defined by straight walls leading to a torus portion, said torus portion being an enlarged portion with a diameter superior to the diameter of the cylindrical portion.

The method according to this embodiment is advantageously modular: the user is free to design a specific pattern of recesses depending on the use of the sensor device. The array can comprise low or high ratio of protrusions depending on the required adhesion force of the sensor device. The user can also designe the pattern based on the relief or structure of the skin to be contacted and adapt the ratio of protrusion depending thereof.

In one embodiment, the walls and the feet of each recess are etched by two orthogonal methods, preferably photolithography for the walls and chemical etching for the feet. In this embodiment, the etchings steps of the walls and the feet are independent allowing an improved control of each of the etching steps.

In an embodiment, the method further comprises:
- Depositing a photosensitive layer on the substrate, said photosensitive layer being dissolvable after exposure to a determined radiation and subsequently treated with a developer solution for photosensitive layer;
- Fabricating the mould by :
   ∘ Etching away selectively the photosensitive layer to provide the cylindrical portion of the recess; and
   ∘ Etching away selectively the substrate to provide the torus portion of the recess;

The recess comprising the cylindrical portion and the torus portion are engraved by two successive etching steps in orthogonal directions: the cylindrical portion is etched in a direction perpendicular or substantially perpendicular to the plan of the mould. The torus portion is etched by engraving the walls perpendicularly with respect of the walls of the cylindrical portion, i.e. in a direction parallel or substantially parallel to the plan of the mould.

Preferably, the torus portion is etched by chemical etching by introducing a chemical etching composition between the walls of the recess. The parameters of the chemical etching step are adjusted by the user depending on the diameter of the feet or the protrusions required. For instance, the parameters comprise the composition of the etching chemical, duration of the etching reaction etc...Particularly, it has been found that the diameter of the feet depends of the duration of the chemical etching.

In one embodiment, the method comprises:
- Depositing successively either a conductive material into the recesses and subsequently a non-conductive material, or a non-conductive material and subsequently a conductive material, said non-conductive material surrounding the conductive material;

The conductive material and the non-conductive material are generally fluid material that changes it viscosity, with temperature, light, chemical reaction for instance. Preferably, the fluid material is deposited into the mould and subsequently cured to provide the conductive or non-conductive portion in solid state.

The conductive material or non-conductive material may be a fluid that is deposited or poured. The conductive material or non-conductive material may be a paste that needs to be flattened by mechanical pressure. The conductive material can also be a suspension of granular particles and/or a colloid (which are depending on the size of the particles).

In one embodiment, the conductive material in a non cured form (fluid or paste for instance) is deposited into the mould in the area delineating as the conductive area of the sensor device to be moulded. Then the non-conductive material in fluid form is deposited around the conductive area in the non-conductive area. Alternatively, the non-conductive material is deposited in the mould, followed by the conductive material. Then the mould with the conductive and non-conductive material is cured.

In another embodiment, it is possible to cure the conductive material before depositing the non-conductive material, and curing the non-conductive material subsequently, mutatis mutandis when the non-conductive material is introduced prior to the conductive material.

In some embodiment, when the conductive material is in a paste form that needs to be flatten before the deposit of the non-conductive material, it is preferable to deposit first the conductive material and then the non-conductive material.

In an embodiment, the method comprises:
- Providing a first mould for moulding the conductive portion, and a second mould for moulding the non-conductive portion;
- Depositing the conductive material into the first mould and the non-conductive material into the second mould;
- Dissolving the first mould and second mould to release the conductive portion and non-conductive portion;
- Embedded the conductive portion into the non-conductive portion to assemble the sensor device.

In this embodiment, the method is repeated to prepare the conductive portion and a non-conductive portion separately. The conductive portion is embedded into the non-conductive portion and the assembly is subsequently cured. Preferably, at least one of the conductive portion or non-conductive portion is partially cured, so that the conductive or non-conductive material is partially molten. The partially molten portion facilitates the adherence between the two portions. Alternatively, an adhesive layer can be deposited between the cured conductive portion and the cured non-conductive portion, the assembly being subsequently, cured to seal the conductive portion with the non-conductive portion. Thus, the non-conductive and conductive portion can be prepared separately and subsequently assembled.

In one embodiment, the method comprises, prior to the depositing step:
- Defining a pattern on the mould, said pattern defining:
- a conductive area designed for receiving the conductive material; and/or
- a non-conductive area designed for receiving the non-conductive and conductive portion.

For instance, the pattern is defined by a divider also called divider insert placed on the mould before depositing the material (conductive or not) into said mould. The divider delineates the conductive area and the non-conductive area. For instance, the divider is a wall with a circular or polygonal shape. The divider can be based on plastic suitable for moulding conductive and/or non-conductive material. For example, the mould is made of PMMA, polyurethane, silicon, teflon.

In another embodiment, the non-conductive and conductive portion of the sensor device are fabricated are fabricated separately and subsequently assembled. Before the assembly, the conductive portion and/or the non-conductive portion is/are cut out to define the geometries or dimensions depending on the needs. In some cases, it is easier to define the geometries of the conductive or non-conductive portion after demoulding, notably when the required geometries are difficult to fabricate with a divider.

In the present invention, "conductive portion" means a portion made of material suitable for conducting an electrical signal.

In the present invention, "non-conductive portion" means a portion made of material not suitable for conducting an electrical signal, in other word an electrical insulator. Besides, the non-conductive portion can be a thermal insulator, a water insulator.

In the present invention, the sensor device can be an electrode, in other words a pad or a patch designed for contacting the skin to detect an electrical signal. The sensor device can be solely the pad or patch. The sensor device can further be equipped with electronical or electrical elements for conducting the signal, such as electrical wires or plates, active electronics such as recording electronics or elements.

In the present invention, the protrusions have micrometer dimensions: in other words, the height and diameter of the protrusions are in the range of micrometer. For instance:
- the diameter of the cylindrical portion of the protrusion between 1 micrometer and 15 micrometer, preferably 3 to 7, more preferably 5;
- the diameter of the feet is between 3 and 19 micrometer, preferably 5 to 9, more preferably 7 micrometer;
- the height of the cylindrical portion is between 7 and 20 micrometer, preferably 9 to 15 micrometer, more preferably 12 micrometer;
- the height of the feet is between 1 and 5 micrometer, preferably 2 to 4, more preferably 2 micrometer.

These ranges also applies to the extensions.

The embodiments described for the sensor device applied mutatis mutandis to the process according to the invention.

### Brief Description of the Drawings

The invention will be better understood with the aid of the description of an embodiment given by way of example and illustrated by the figures, in which:
Figure 1 shows a section view of a sensor device according to a first embodiment;
Figure 2 shows a section view of a sensor device according to a second embodiment;
Figures 3 to 12 shows a process according to the invention.

### Detailed Description of possible embodiments of the Invention

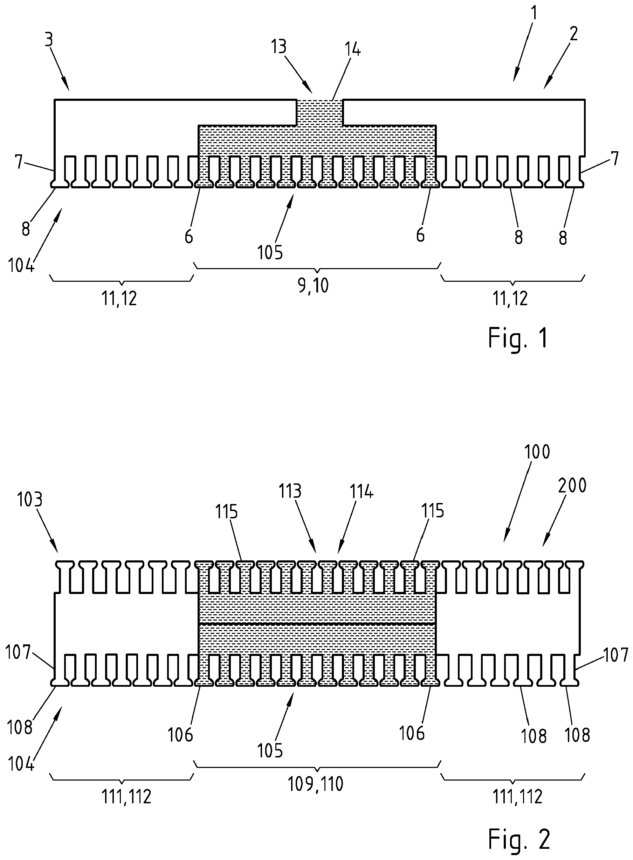

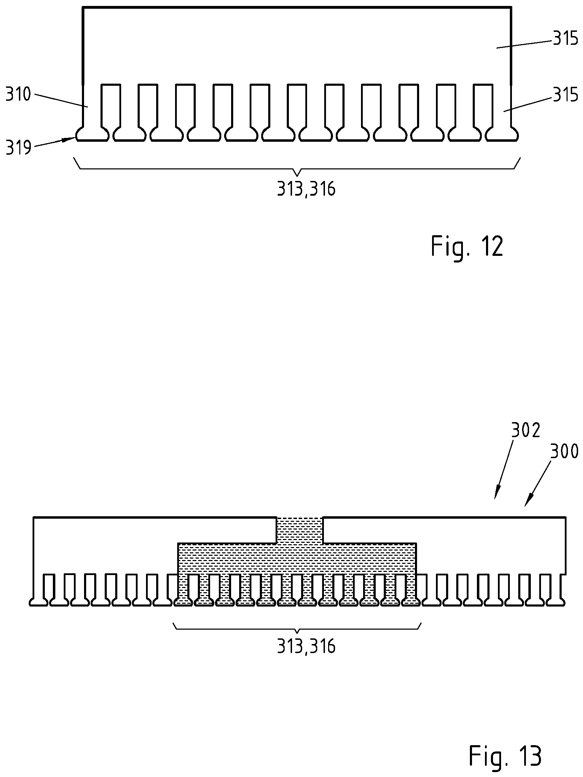

Figures 1 to 12 illustrate some embodiments of the present invention but it is intended that the invention is not limited to these embodiments.

Figure 1 illustrates a sensor device 1 according to a first embodiment. In this first embodiment, the sensor device 1 is an electrode 2 comprising an upper face 3 and a lower face 4.

The electrode 2 is designed for being placed on the skin of a user to detect an electrical signal, for instance for electrocardiography (ECG) i.e. recording the electrical activity if the heart.

The lower face 4 comprises an array of protrusions 5, each protrusions 6 protruding towards the skin of the user when the electrode 2 is placed on the skin. The protrusions 6 have a mushroom shape (also called gecko or grasshopper shape): each protrusion 6 comprises a rod 7 having a cylindrical shape, said attached to the lower face 4 and leading to a feet 8 having an oval cross section. The feet 8 is an enlarged portion of the rod 7 with a diameter superior to the diameter of the rod 7. In the present case, the rod 7 has a diameter of 5 µm, the feet having 7 µm diameter, the protrusion having a total height (feet plus rod) of 12 µm.

The array 5 of the lower face 3 comprises a conductive portion 9 in a conductive area 10 and a conductive portion 11 in a non-conductive area 12. In the present case, the conductive area 10 and the non-conductive area 12 are two concentric circles on the array 5, the conductive area 10 being a central area and the nonconductive area 12 surrounding the conductive area 10.

The electrode 2 further comprises a connector 13 for guiding the signal from the lower face 3 to the upper face 4. In the present embodiment, the connector 13 is a conductive bridge 14 extending from the conductive portion 9 and embedded in the non-conductive portion 11. The conductive bridge 14 is further connected to metallic wires linked to a read out device (not shown in figures) in charge of recording the signal.

Figure 2 show a second embodiment of a sensor device 100. The sensor device 100 is an electrode 101, said electrode 101 comprising an upper face 103 and a lower face 104. The lower face 104 is similar to the lower face 4 of the electrode 2 of the first embodiment and comprises
- an array of protrusions 105, each protrusion 106 having a rod 107 and a feet 108;
- the lower face 104 further comprising a conductive portion 109 in a conductive area 110, and a non-conductive portion 111 in a non-conductive area 112.

In the second embodiment, the electrode 102 comprises a connector 113, said connector 113 being a connector pad 114. The connector pad 114 has a plurality of extensions 115 extending from the connector pad 114 in a direction opposite to the skin when the electrode 100 in placed on the skin. The extensions 115 also act as interlocking means for interlocking the electrode 100 with metallic wires or plates (not shown in figures) linked to a read out device (not shown in figures) in charge of recording the signal or a stimulating device in case the electrode is used for stimulating.

In the present embodiment, the upper face 103 with the connector pad 114 and the lower face 104 with the conductive portion 109 are mirror image of each other.

The electrode 102 is a double sided electrode, whereas the electrode 2 in the first embodiment was a single sided electrode. The upper side 103 of the electrode 102 comprises interlocking means 113 for interlocking the device with a read out device (not shown in figures) several extensions

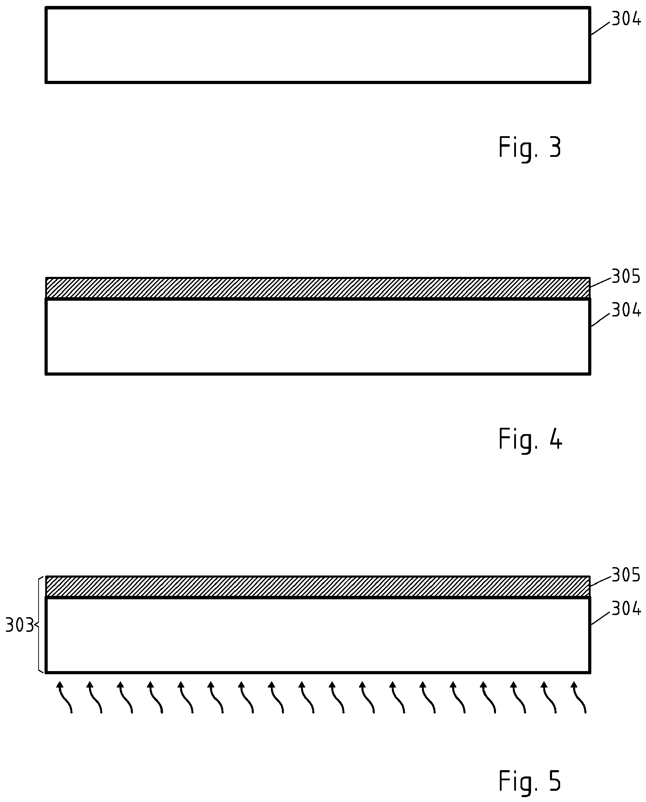

A method according to the present invention for manufacturing a sensor device 300 according to a third embodiment is represented in figures 3 to 12, the manufactured sensor device 300 being an electrode 302.

A sacrificial layer 303 is deposited in a flat base material 304 by homogenous spin coating, as shown in figures 3 and 4. Typically, the flat base material is a wafer. The sacrificial layer 303 has a high adhesion to the base material 304 and is not photosensitive but can be dissolved in a chemical developer solution. In the present embodiment, the base layer is a silicon wafer, but glass wafer or any other planar surface element that provides sufficient adhesion for sacrificial layer deposit and that does not get etched by the developer used for the sacrificial layer can be used. The sacrificial layer 303 is a polydimethlyglutarimide (PMGI) derivative, but other polymeric thin film that get chemically etched by the determined developer solution can be used.

The sacrificial layer 303 is then soft baked, i.e. carefully heat treated, on a hot plate (see figure 5).

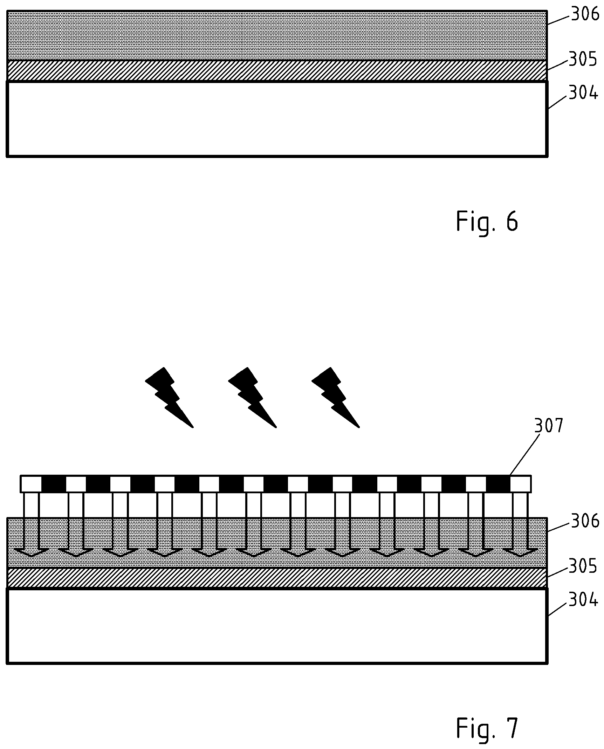

A photosensitive photoresist layer 306 is deposited on the sacrificial layer 305 via homogenous spin coating as shown in figure 6. The sacrificial layer 303 and the photoresist layer 306 can be etched by orthogonal methods so as be selectively engraved. For instance :
- A) The sacrificial layer 303 is not photosensitive and designed for being chemically etched, whereas the photoresist layer 306 is photosensitive; or
- B) Both the sacrificial layer 303 and the photoresist layer 304 are photosensitive, but present very different rates of dark erosion and do not mix when put on top of each other;

In the present embodiment, the method illustrated in figures 3 to 12 corresponds to option A.

In the present embodiment, the substrate that will be engraved into a mould is a bilayer and comprises a sacrificial layer and a photoresist layer. Alternatively, it is possible to use a monolayer substrate, for instance a unique sacrificial layer or a unique photoresist layer.

The photoresist 306 is exposed to 365 or 405 nm light through a mask 307 letting light pass on selected segments only, as shown in figure 7. Alternatively, a laser can also be used. In the present embodiment, a positive photoresist 306 is used so that the exposed segment become solvable in a specific developer solution. Alternatively, a negative photoresist can be used with a reverse process.

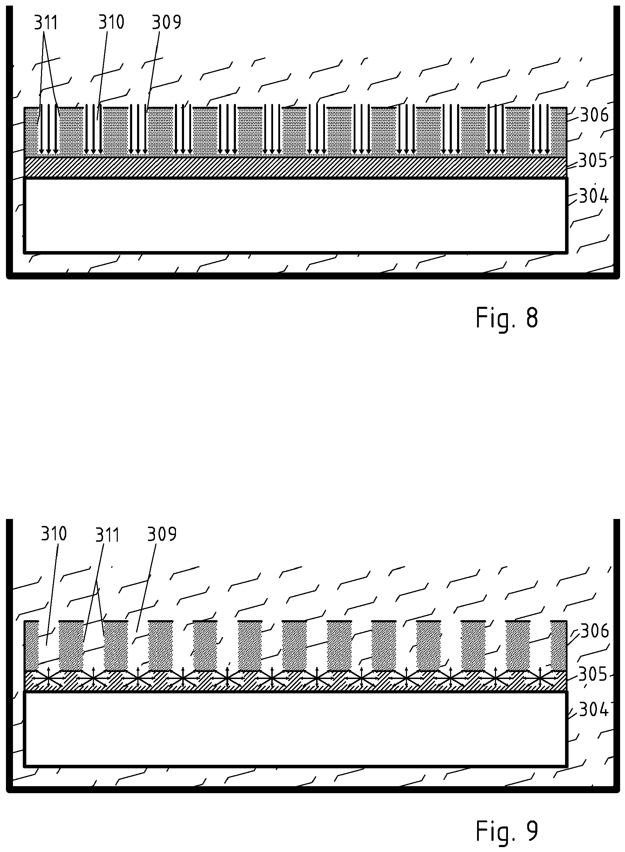

The exposed segments are subsequently dissolved in a developer solution to provide an array of recesses 308, each recess 309 comprising a cylindrical portion 310 with straight walls 311 etched in the photoresist layer 306, as shown in figure 8. The cylindrical portion 310 of the mould is used to mould the feet of the protrusion.

Each recess 309 further comprises a torus portion 319 in the bottom of the recess 309, said torus portion 319 being received in the sacrificial layer 305. The torus portion 319 of the mould is used to mould the feet of the protrusion. To that end, the sacrificial layer 305 is chemically etched by immersion in a developer solution bath for etching specially the sacrificial layer 305, as shown in figure 9. In the present embodiment, the developer solution for the sacrificial layer 305 is different from the one used to dissolve the exposed segment of the photoresist 306 in the previous step, but it is possible to use a single developer solution for performing these steps.

During the chemical etching of the sacrificial layer, the diameter of the bottom of the cylindrical portion 310 extends radially, i.e. in a direction y, to create the torus portion 319, the torus portion 319 being an enlarged portion of the cylindrical portion of the recess. The dimension of the torus portion 319 depends notably on the immersion duration: the user adjusts the immersion duration depending on the required diameter of the torus portion 319. In the present case, the developer is a basic buffered metal containing solution of KOH. The immersion lasts between 15 s to 7 min.

Then, the electrode in process is removed from the developer bath and subsequently washed with water and dried off to clear the array 308, as shown in figure 9.

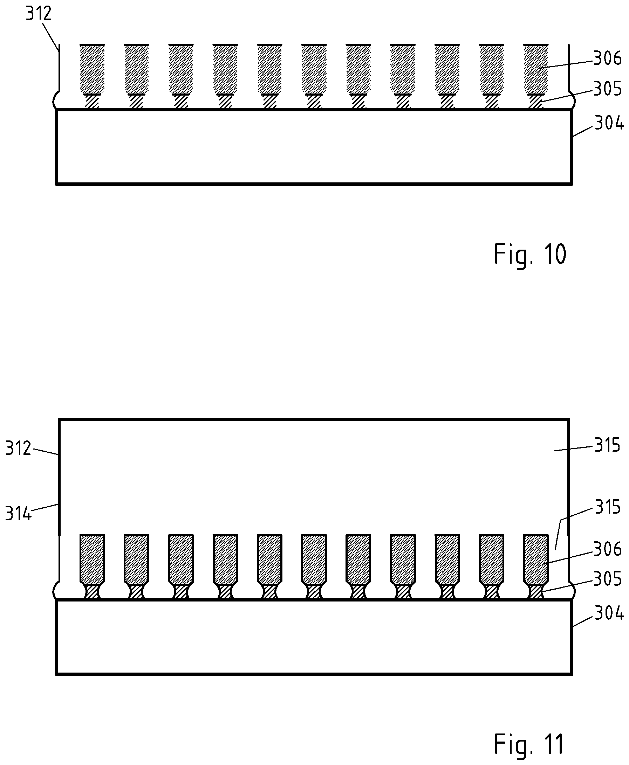

A divider 312 is placed on the array 308 to delineate the conductive area 313. In the present case, the divider 312 is formed by a circular wall 314 made of plexiglas. Then, a conductive material 315 is deposited into the conductive area 313 as shown in figure 10. In the present embodiment, the conductive material 315 is based on non-conductive material mixed with conductive microparticles: non-conductive polydimethlsioxane (PDMS or ecoflex) mixed with conductive silver microparticles from 1.5 to 3.5 µm.

Subsequently, the wafer is cured to solidify the conductive material 315.

After curing, the mould made of photoresist layer 306 and the sacrificial layer 305 is dissolved in a 1 Sensor device
determined developer solution so that the conductive material 315 structured in the array of recess 308 detaches from the flat base material 304. The flat base 304 is then reusable. At this stage of the process, the electrode 302 solely comprises the conductive portion 316 made of conductive material 315.

The method illustrated in figures 3 to 11 is repeated mutatis mutandis to provide the non-conductive portion of the electrode. To that end, a second mould designed to mould a non-conductive portion surrounding the manufactured conductive portion is fabricated by using non-conductive material and a suitable divider, to provide a non-conductive portion 317 of the electrode 302.

The conductive portion 318 and the conductive portion 316 are assembled and cured to provide the electrode 302.

### References of the figures

- 2: Electrode
- 3: Upper face
- 4: Lower face
- 5: Array of protrusion
- 6: Protrusion
- 7: Rod
- 8: Feet
- 9: Conductive portion
- 10: Conductive area
- 11: Non-conductive portion
- 12: Non-conductive area
- 13: Connector
- 14: Conducting bridge

- 100: Sensor device
- 102: Electrode
- 103: Upper face
- 104: Lower face
- 105: Array of protrusion
- 106: Protrusion
- 107: Rod
- 108: Feet
- 109: Conductive portion
- 110: Conductive area
- 111: Non-conductive portion
- 112: Non-conductive area
- 113: connector
- 114: Connector pad
- 115: Extension

- 300: Sensor device
- 302: Electrode
- 303: Substrate
- 304: Base
- 305: Sacrificial layer

- 306: Photoresist layer
- 307: Mask
- 308: Array of recesses
- 309: Recess
- 310: Cylindrical portion
- 311: Wall
- 312: Divider
- 313: Conductive area
- 314: Circular wall of the divider
- 315: Conductive material
- 316: Conductive portion
- 317: Non-conductive portion
- 318: Conductive portion
- 319: torus portion

## Claims

1. Sensor device (1, 100, 200, 300) designed for contacting the skin of a user and detecting an electrical signal, the device (1, 100, 200, 300) comprising:
- two main faces, an upper face (3, 103) and a lower face (4, 104) opposed to the upper face (3, 103), the lower (4, 104) face being designed for being placed toward the skin, the upper face (3, 103) being designed for transmitting the signal,
- the lower face (4, 104) comprising an array of protrusions (5, 105) with micrometre dimensions, said protrusions (6, 106) protruding from the lower face (4, 104) toward the skin when the sensor (1, 100, 200, 300) is placed on the skin,
**characterized in that** the array (5, 105) comprises a conductive portion (9, 109, 316) and a non-conductive portion (11, 111, 318), said non-conductive portion (11, 111, 318) surrounding said conductive portion (9, 109, 316), so that when the sensor (1, 100, 200, 300) is placed on the skin, the non-conductive portion (11, 111, 318) allows maintaining the contact between the conductive portion (9, 109, 316) and the skin to ensure that the conductive portion (9, 109, 316) is capable of detecting the signal.

2. Sensor device (1, 100, 200, 300) according to claim 1, wherein the protrusions (6, 106) comprise a rod (7, 107) attached to the lower face, and a feet (8, 108) at the distal end of said rod (7, 107), the feet (8, 108) having a diameter superior to the diameter of the rod (7, 107).

3. Sensor (1, 100, 200, 300) device according to any one of claims 1 or 2, wherein the array (5, 105) comprises a central conductive portion (9, 109, 316) surrounded by a peripheral non-conductive portion (11, 111, 318).

4. Sensor device (1, 100, 200, 300) according to any one of claims 1 to 3, wherein the upper face (3, 103) further comprises a connector (13, 113) connected with the lower face (4, 104), said connector (13, 113) allowing guiding the signal from the lower face (3, 103) to the upper face (4, 104).

5. Sensor device (1, 100, 200, 300) according to any one of claims 1 to 4, wherein the upper face (4, 104) comprises interlocking means (115) designed for interlocking the sensor device (1, 100, 200, 300) with an external device, for instance a read out device.

6. Sensor device (1, 100, 200, 300) according to claim 5, wherein the interlocking means (115) comprise a plurality of extensions (115) extending from the upper face (4, 104).

7. Sensor device (1, 100, 200, 300) according to any one of claims 1 to 6, wherein the conductive portion (11, 111, 318) comprises conductive nanoparticles or conductive microparticles, preferably with particles size between 0.1 nm and 10 µm, preferably 0.1 nm and 3500 nm, for example silver (Ag) nanoparticles, silver (Ag) microparticles.

8. Sensor device (1, 100, 200, 300) according to any one of claims 1 to 7, wherein at least part of the sensor device, in particular the conductive portion and/or the non-conductive portion, is made of soft material, for example a soft material with a softness in the range of the human skin.

9. Method for manufacturing a sensor device (1, 100, 200, 300) designed for contacting the skin of a user and detecting an electrical signal, the method comprising:
- i) Providing a mould for moulding the sensor device (1, 100, 200, 300), the mould being constituted with a substrate (303) designed for being dissolvable in a determined developer solution, the substrate comprising an array of recesses (308) etched in said substrate (303);
- ii) Depositing a conductive material (315) and/or a non-conductive material into the recesses (309) of the array (308);
- iii) Dissolving the mould with the determined developer solution to release the sensor device (1, 100, 200, 300).

10. Method according to claim 9, the method further comprises :
- Fabricating the mould by etching away selectively the substrate (303) to etch the recesses (309), each recess (309) comprises a cylindrical portion (310) attached the lower face and defined by straight walls (311) leading to a torus portion (319), said torus portion (319) being an enlarged portion with a diameter superior to the diameter of the cylindrical portion (311).

11. Method according to the preceding claim, wherein the cylindrical portion (311) and the torus portion (319) of each recess (309) are etched by two orthogonal methods, preferably photolithography for the cylindrical portion (311) and chemical etching for the torus portion (319).

12. Method according to any one of claims 1 to 11, wherein the method further comprises:
- Depositing a photosensitive layer (306) on the substrate (303), said photosensitive layer (306) being dissolvable after exposure to a determined radiation and subsequently treated with a developer solution for photosensitive layer (306);
- Fabricating the mould by :
∘ Etching away selectively the photosensitive layer (306) to provide the cylindrical portion (310) of the recess (309) and
∘ Etching away selectively the substrate (303) to provide the torus portion (319) of the recess (309) ;

13. Method according to any one of claims 10 to 12, wherein the method comprises:
- Depositing into the mould successively either a conductive material (315) into the recesses (309) and subsequently a non-conductive material, or a non-conductive material and subsequently a conductive material (315), said non-conductive material surrounding the conductive material (315);

14. Method according to any one of claims 10 to 13, wherein the method comprises:
- Providing a first mould for moulding the conductive portion, and a second mould for moulding the non-conductive portion;
- Depositing the conductive material (315) into the first mould and the non-conductive material into the second mould;
- Dissolving the first mould and second mould to release the conductive portion (11, 111, 318) and non-conductive portion (9, 109, 316);
- Embedded the conductive portion (11, 111, 318) into the non-conductive portion (9, 109, 316) to assemble the sensor device (1, 100, 200, 300).

15. Method according to any one of claims 10 to 14, wherein the method comprises, prior to the depositing step:
- Defining a pattern on the mould, said pattern defining:
∘ a conductive area (10, 110, 313) designed for receiving the conductive material (315); and/or
∘ a non-conductive area (12, 112), designed for receiving the non-conductive material.
